# EUROPÄISCHE PATENTANMELDUNG

(11) **EP 1 764 077 A2**
(43) Veröffentlichungstag der Anmeldung: **21.03.2007**
(21) Anmeldenummer: 06019479.2
(22) Anmeldetag: 18.09.2006
(51) Int. Cl.: A61K 8/02, A61K 8/81, A61Q 19/10

(54) **Wattescheibe zum Reinigen und Peeling der Haut**

(30) Priorität: 16.09.2005 DE 202005014927 U
(71) Anmelder: W. Pelz GmbH & Co. KG, 23812 Wahlstedt/Holstein (DE)
(72) Erfinder: Seiss, Willi Dr., D-24537 Neumünster (DE); Bark, Andreas Dr., D-22397 Hamburg (DE)
(74) Vertreter: Siemons, Norbert

(57) **Zusammenfassung**

Wattescheibe zum Reinigen und Peeling der Haut umfassend mindestens eine Faserschicht und einen abrasiven, mit der Faserschicht verbundenen Bindemittelauftrag an mindestens einer Außenseite.

## Beschreibung

Die Erfindung bezieht sich auf eine Wattescheibe zum Reinigen und Peeling der Haut.

Wattescheiben (auch "Pad", "Wattepad", "Kosmetikpad" oder "Wattetupfer" genannt) werden in der Kosmetik zum Entfernen von pastösen oder flüssigen Mitteln, beispielsweise beim Abschminken, eingesetzt. Zum Peeling geeignete Kosmetikpads weisen zumindest eine rauhe Außenseite auf, die zum "Peeling", d.h. zum Entfernen von abgestorbenen Hautschuppen, verwendet wird. Die andere Außenseite ist meistens in herkömmlicher Weise ausgeführt und zum Entfernen von pastösen oder flüssigen Mitteln nutzbar.

Aus der EP 1 310 226 A2 ist ein kosmetisches Wattepad bekannt, das an beiden Oberseiten durch Wasservernadelung erzeugte Feinrillen und an zumindest einer Oberseite ein Profil aus Rippen, flächigen Einsenkungen und/oder Breitrillen aufweist. Diese durch Wasservernadelung erzeugten Profile erhöhen die Festigkeit der Wattescheibe gegen mechanische Beanspruchung, ergeben eine Depotwirkung für pastöse Substanzen und verhindern insbesondere beim Abschminken unerwünschtes Fusseln. Die Rippen, flächigen Einsenkungen und/oder Breitrillen haben eine wischblattähnliche Abstreifwirkung und können zum Peeling genutzt werden.

Aus der WO 2004/007826 A2 ist ein zur Reinigung und Peeling der Haut geeignetes Kosmetikpad mit einer Faserschicht bekannt, wobei zumindest eine Seite des Kosmetikpads oberflächenrauh ausgebildet ist, indem dort ein partikuläres Schleifmittel aufgesintert ist. Beim Einsatz auf der Haut lösen sich die für den Peelingeffekt verantwortlichen Partikel von der Oberfläche ab, was als störend empfunden wird.

Ferner sind aus dem Markt Wattepads zum Abschminken und sanften Peeling der Haut bekannt, die unter der Oberfläche eingebundene Luffafasern haben. Die Wattepads haben einen nur mäßigen Peelingeffekt.

Die EP 1 283 019 A1 offenbart einen texturierten Artikel, der auf zumindest einer Außenseite erhabene Elemente hat, wobei der Artikel für die Haut- bzw. Haarpflege vorteilhaft ist und die erhabenen Elemente aus Beschichtungen von Hotmelt, umfassend ein Olefin-Polymer, ein Block-Copolymer oder eine Kombination davon gebildet sind.

Das Hotmelt wird im schmelzflüssigen Zustand aufgebracht und erkaltet sogleich bei Kontakt mit dem Substrat, ohne in dieses einzudringen. Das Hotmelt hat nur eine schwache Verbindung zu dem Substrat und kann sich leicht bei Anwendung des Wattepads lösen. Außerdem kann das Hotmelt z.B. in Form von Fäden auf die Oberfläche des Substrates aufgebracht werden. Ferner kann das Hotmelt im Schlitzdüsenverfahren direkt auf das Substrat aufgebracht werden. Dabei tritt das flüssige Hotmelt aus einem Schlitz aus und wird auf das darunter laufende Substrat als Film mitgezogen und aufgezogen. Außer einem geschlossenen Film sind auch gerade Streifen in Maschinenlaufrichtung grundsätzlich möglich. Nicht möglich sind hingegen geschwungene Flächen oder gar Figuren.

Davon ausgehend liegt der Erfindung die Aufgabe zugrunde, eine Wattescheibe zum Reinigen und Peeling der Haut zu schaffen, die ein Peeling ermöglicht, ohne daß sich in störendem Maße Partikel von der Oberfläche lösen.

Die Aufgabe wird durch eine Wattescheibe mit den Merkmalen des Anspruches 1 gelöst. Vorteilhafte Ausgestaltungen der Wattescheibe sind in den Unteransprüchen angegeben.

Die erfindungsgemäße Wattescheibe zum Reinigen und Peeling der Haut umfaßt mindestens eine Faserschicht und einen abrasiven, mit der Faserschicht verbundenen Bindemittelauftrag an mindestens einer Außenseite.

Die erfindungsgemäße Wattescheibe weist an mindestens einer Außenseite einen abrasiven Bindemittelauftrag auf, der mit einer Faserschicht verbunden ist. Der Bindemittelauftrag wird durch Applizieren einer Emulsion bzw. Dispersion von Tröpfchen bzw. Teilchen von mindestens einem Polymer in einem Lösungsmittel auf die Faserschicht aufgebracht. Die Emulsion bzw. Dispersion dringt zumindest in die oberflächennahen Bereiche der Faserschicht ein. Nach Verdunstung bzw. Verdampfung des Lösungsmittels bilden die polymeren Teilchen einen mehr oder weniger zusammenhängenden Polymerfilm bzw. Kunststoffilm. Dieser Bindemittelauftrag ist mechanisch und/oder durch Adhäsionskräfte mit der Faserschicht verbunden. Die Verbindung des Bindemittelauftrags mit der Faserschicht ist deshalb besonders fest. Aufgrund der Verfestigung der Wattescheibe an der mindestens einen Außenseite kann die Außenseite für das Peeling herangezogen werden. Dabei kann der Bindemittelauftrag so eingestellt sein, daß er besonders abrasiv wirkt, insbesondere durch Auswahl geeigneter Polymere. Beim Peeling werden durch den Bindemittelauftrag Hautschuppen und andere Hautunreinheiten abgetragen. Die abrasive Wirkung des Bindemittelauftrages beruht auf einer Rauhigkeit der Oberfläche des Bindemittelauftrages und/oder auf der Verfestigung bzw. verringerten Weichheit des Wattepads im Bereich des Bindemittelauftrages, der einer Verformung des Wattepads einen größeren Widerstand entgegensetzt, als eine bindemittelfreie Außenseite. Aufgrund der besonders festen Verbindung des Bindemittelauftrages mit der Faserschicht löst sich der Bindemittelauftrag nicht von der Wattescheibe. Der mit der Faserschicht verbundene Bindemittelauftrag sondert beim Peeling keine harten Körner oder andere störende Partikel ab. Ferner ist vorteilhaft, daß der Bindemittelauftrag ein Ablösen von Fasern der Faserschicht gerade an den beim Peeling besonders belasteten Stellen wirksam verhindert. Die Anwendung wird als angenehmer als bei den Wattepads mit aufgesinterten Partikeln empfunden. Die mit dem Bindemittelauftrag versehene Außenseite kann Hautunreinheiten, Schminke und dgl. anlagern bzw. absorbieren. Die beim Peeling von der Haut abgelösten Substanzen werden somit in demselben Arbeitsgang von der Haut entfernt und nicht nur über die Haut verteilt. Ferner kann der Bindemittelauftrag die Fasern und damit die Außenseite der Wattescheibe partiell verfestigen und damit die Gebrauchseigenschaften der Wattescheibe erhöhen, zusätzlich zu der abrasiven Eigenschaft.

Unter einem abrasiven Bindemittelauftrag wird ein Bindemittelauftrag an einer Außenseite einer Faserschicht einer Wattescheibe verstanden, der der Wattescheibe die Fähigkeit verleiht, Hautschuppen oder andere Hautunreinheiten von der Haut in einem deutlich stärkeren Ausmaß abzutragen, als eine Wattescheibe, die sich von der vorbeschriebenen allein dadurch unterscheidet, daß die Außenseite der Faserschicht bindemittelfrei ist. Dies zeigt sich beispielsweise darin, daß die mit dem Bindemittelauftrag versehene Wattescheibe von einer Oberfläche, an die Feststoffpartikel anhaftend angelagert sind, bei einer bestimmten Anzahl (z.B. 5) Wischvorgängen eine erheblich größere Menge (z.B. 50 % mehr) der Feststoffpartikel abträgt, als eine Wattescheibe, die an der Außenseite bindemittelfrei ist.

Die Erfindung bezieht Ausgestaltungen der Wattescheiben ein, die auf beiden Außenseiten mit einem Bindemittelauftrag versehen sind, die übereinstimmend oder unterschiedlich ausgeführt sein können, letzteres, um verschiedene Peelingeigenschaften zur Verfügung zu stellen. Ferner bezieht die Erfindung Wattescheiben ein, die den Bindemittelauftrag nur auf einer Seite aufweisen und auf der anderen Seite eine vom Bindemittelauftrag freie Faserschicht haben. Bei dieser Ausgestaltung kann die vom Bindemittelauftrag freie Außenseite für eine intensive Nachreinigung der Haut genutzt werden.

Ferner bezieht die Erfindung Wattescheiben ein, die gemäß EP 1 310 226 A2 ausgeführt sind. Dies gilt insbesondere für die Zusammensetzung aus Faserstoffen, die Wasserstrahlvernadelung und das Profil aus Rippen, flächigen Einsenkungen und/oder Breitrillen. Die diesbezüglichen Inhalte der EP 1 310 226 A2 werden durch Bezugnahme in vorliegende Anmeldung einbezogen. Gemäß EP 1 310 226 A2 ausgestaltete Wattescheiben werden durch Bindemittelauftrag auf zumindest eine der beiden Außenseiten zu einer erfindungsgemäßen Wattescheibe.

Gemäß einer Ausgestaltung weist die Außenseite den Bindemittelauftrag in mindestens einem Teilbereich und mindestens einem weiteren, vom Bindemittelauftrag freien Teilbereich auf.

Durch den nur partiellen Bindemittelauftrag an der Außenseite des Wattepads wird ein besonders effektives Peeling ermöglicht, weil der Wechsel verfestigter und nicht bzw. weniger verfestigter Bereiche eine besonders gute Abtragswirkung für Hautunreinheiten aufweist. Außerdem können die vom Bindemittelauftrag freien Teilbereiche besser Hautunreinheiten, Schminke und dgl. anlagern bzw. absorbieren. Die beim Peeling von der Haut abgelösten Substanzen werden somit effektiver in demselben Arbeitsgang von der Haut entfernt.

Der Bindemittelauftrag kann auf verschiedene Weise über die Außenseite verteilt sein. Gemäß einer Ausgestaltung ist der Bindemittelauftrag entlang mindestens einer kreisförmigen und/oder wellenförmigen und/oder dreiecksförmigen und/oder schuppenförmigen und/oder geraden und/oder einander kreuzenden Linien oder Bahnen angeordnet. Gemäß einer Ausgestaltung bildet der Bindemittelauftrag ein Logo und/oder eine sonstige Kennzeichnung und/oder mindestens ein Ornament.

Gemäß einer Ausgestaltung weist der Bindemittelauftrag eine andere Farbe als die Faserschicht auf. Durch unterschiedliche Farben kann die Funktion der mit einem Bindemittelauftrag versehenen Außenseite der Wattescheibe verdeutlicht werden. Außerdem können hierdurch dekorative Muster verwirklicht werden.

Gemäß einer weiteren Ausgestaltung ist der Bindemittelauftrag als wässrige Emulsion oder Dispersion polymerer Tröpfen oder Teilchen aufgebracht. Gemäß dieser Ausgestaltung ist der Bindemittelauftrag unter Verwendung von Wasser als Lösungsmittel für das mindestens eine Polymer aufgebracht. Das Wasser wird verdunstet bzw. verdampft, so daß das innig mit der Faserschicht verbundene Polymer an der Wattescheibe verbleibt.

Gemäß einer Ausgestaltung enthält der Bindemittelauftrag ein Acrylpolymer. Acrylpolymere sind als mit einer Faserschicht verbindbarer Bindemittelauftrag besonders gut geeignet. Bevorzugt werden den Acrylpolymeren abrasive Wirkstoffe zugefügt, um eine abrasive Wirkung zu erreichen. Die abrasiven Zusatzstoffe werden durch den Bindemittelauftrag so eingebunden, daß beim Peeling keine störenden Partikel freigesetzt werden. Das Acrylpolymer wird bevorzugt als wässrige Emulsion bzw. Dispersion auf die Faserschicht aufgebracht.

Ein geeignetes Acrylpolymer wird von der Firma Celanese, Bridgewater, NJ 08807 unter der Produktbezeichnung "NACRYLIC^{™} ABX-30" vertrieben. Das ABX-30 ist selbstvernetzendes Bindemittel, das nach Trocknung bei höheren Temepraturen (über 100°) vernetzt (durch chemische Reaktion) und danach wasserunlöslich wird. Der Bindemittelauftrag wird folglich nicht durch Wasser wieder angelöst, was bei einer Wattescheibe zum Reinigen und Peeling unerwünscht wäre.

Für die Herstellung des Bindemittelauftrages kommen insbesondere Emulsionen bzw. Dispersion auf Basis der folgenden Polymere in Betracht:
- Polyvinylester, insbesondere Vinylacetat-Homopolymeren (mit und ohne Weichmacher)
- Polyvinylacetate, insbesondere Vinylacetat-Maleinsäureester-Copolymeren
- Vinylacelat-Versatat-Copolymeren
- Vinylacelat-Acrylat- Copolymere, z.B. Vinylacelat/Alkylacrylat- Copolymeren
- Vinylacelat-Olefin-Copolymeren, z.B. Vinylacelat/Ethylen- Copolymeren
- Ethylen-Vinylacetat-Acrylat-Terpolymeren
- Vinylacetat-Vinylchlorid-Ethylen-Acrylat-Copolymeren
- Polyacrylsäureestern
- Reinacrylatdispersionen
- Polyurethanmodifizierte Reinacrylaten
- Polyurethan-Hybrid-Dispersionen
- Styrol-Butadien-Copolymeren
- Styrol-Acrylat-Copolymeren
- Styrol-Acrylnitril-Copolymeren
- Acrylnitril-Butadien-Copolymeren
- Acrylnitril-Butadien-Styrol-Terpolymeren
- Syntheselatex auf Basis von Butadien

Es handelt sich dabei bevorzugt um wässrige Emulsionen bzw. Dispersionen.

Gemäß einer Ausgestaltung enthält der Bindemittelauftrag Peelingkörper und/oder Wirkstoffe. Die abrasive Wirkung des Bindemittelauftrages kann durch Peelingkörper verstärkt werden. Hierbei kann es sich insbesondere um synthetische oder natürliche Peelingkörper handeln. Die natürlichen Peelingkörper bzw. Peelingsubstanzen sind z.B. heruntergemahlene Schalen von Früchten, wie Cranberry. Bei den Wirkstoffen handelt es sich z.B. um hautberuhigende und/oder entzündungshemmende Wirkstoffe oder um chemische Peelingwirkstoffe. Die Peelingkörper und/oder Wirkstoffe und/oder Farbstoffe bzw. Farbpigmente sind insbesondere mechanisch und/oder durch Adhäsionskräfte in dem Bindemittelauftrag verankert.

Gemäß einer Ausgestaltung ist der Bindemittelauftrag im wesentlichen in die gesamte Tiefe der Faserschicht eingedrungen und mit dieser verbunden. Der Bindemittelauftrag kann dann zugleich der Verfestigung der gesamten Faserschicht bzw. des gesamten Wattepads dienen. Auch kann der Bindemittelauftrag mehrere aufeinanderliegende Faserschichten zusammenhalten, die den Wattepad bilden, ggf. zusammen mit weiteren Maßnahmen der Verbindung der Faserschichten wie Wasserstrahlvernadelung und/oder mechanische Vernadelung und/oder Verkleben. Gemäß einer Ausgestaltung ist der Bindemittelauftrag nur in oberflächennahen Bereichen in die Faserschicht eingedrungen und mit dieser verbunden. Hierdurch kann eine besonders abrasive Oberfläche bzw. Außenseite des Bindemittelauftrages erreicht werden.

Gemäß einer weiteren Ausgestaltung weist das Wattepad einen im wesentlichen in die gesamte Tiefe der Faserschicht eingedrungenen und mit dieser verbundenen Bindemittelauftrag und einen weiteren, nur in oberflächennahen Bereichen der Faserschicht eingedrungenen und mit dieser verbundenen Bittelmittelauftrag auf. Der erstgenannte Bindemittelauftrag dient der Verfestigung der Faserschicht bzw. des gesamten Wattepads. Der zweitgenannte Bindemittelauftrag dient einer besonderen Verfestigung der Außenseite im Hinblick auf die angestrebte Abrasivität.

Beispielsweise wird eine Wattescheibe aus mindestens einer Faserschicht aus einem wasserstrahlvernadelten Nonwoven aus Baumwollfaser und/oder Viskosefasern und/oder Kunstfasern im wesentlichen über die gesamte Tiefe mit einem Bindemittelauftrag auf der Basis eines Acrylpolymers versehen. Ein zusätzlicher Bindemittelauftrag wird nur in den oberflächennahen Bereichen zumindest einer Außenseite aufgebracht.

Gemäß einer weiteren Ausgestaltung ist der Bindemittelauftrag im Druck- und/oder Sprüh- und/oder Schaumauftragsverfahren aufgebracht. Die genannten Auftragsverfahren können sowohl für einen Bindemittelauftrag im wesentlichen in der gesamten Tiefe der Faserschicht als auch für einen Bindemittelauftrag in oberflächennahen Bereichen der Faserschicht herangezogen werden.

Gemäß einer Ausgestaltung ist der Bindemittelauftrag an der Oberfläche bzw. Außenseite strukturiert. Durch die strukturierte Oberfläche kann die Abrasivität des Bindemittelauftrages beeinflußt werden. Sie ist insbesondere bei Anwendung der vorerwähnten Auftragsverfahren beeinflußbar.

Gemäß einer Ausgestaltung weist die Wattescheibe ein Flächengewicht von 160 bis 320 g/m² auf. Gemäß einer weiteren Ausgestaltung weist es ein Flächengewicht von 220 bis 250 g/m² auf.

Gemäß einer Ausgestaltung weist der Bindemittelauftrag ein Flächengewicht von 2 bis 30 g/m² auf. Hierdurch wird die Ausdehnung des Bindemittelauftrages auf die oberflächennahen Bereiche der Faserschicht beschränkt, insbesondere bei den oben genannten Flächengewichten des Pads. Gemäß einer weiteren Ausgestaltung weist der Bindemittelauftrag ein Flächengewicht von 5 bis 10 g/m² auf.

Der Bindemittelauftrag bedeckt die Außenseite der Faserschicht nur teilweise. Gemäß einer Ausgestaltung bedeckt der Bindemittelauftrag 5 bis 50 % der Außenseite. Hierdurch wird ein für das Peeling besonders vorteilhaftes Verhältnis abrasiver Bereiche und ad- bzw. absorbierender Bereiche auf der Außenseite erreicht.

Gemäß einer Ausgestaltung bedeckt der Bindemittelauftrag 10 bis 30 % der Außenseite.

Die Faserschicht kann verschiedene Beschaffenheit und Zusammensetzungen aufweisen. Einbezogen sind insbesondere Ausführungen aus gebleichten und/oder ungebleichten Baumwollfasern und/oder Viskosefasern und/oder Kunstfasern.

Ferner können die Fasern auf verschiedene Weise zu einer Faserschicht verbunden sein. Einbezogen ist beispielsweise eine Ausführung aus einem trockengelegten Vlies. Gemäß einer Ausgestaltung ist die Faserschicht wasserstrahlvernadelt und/oder mittels Nadeln mechanisch vernadelt. Gemäß einer Ausgestaltung umfaßt die Faserschicht thermoverfestigte Kunststoffasern.

Die Wattescheibe kann grundsätzlich eine beliebige Form aufweisen, z.B. oval, kreisrund oder rechteckig sein.

Ferner sind in die Erfindung Ausführungen einbezogen, die mehrere Faserschichten derselben oder unterschiedlicher Beschaffenheit aufweisen. Von der Erfindung erfaßt sind außerdem Wattescheiben, die außer mindestens einer Faserschicht eine Schicht anderer Art umfaßt, z.B. ein eingelegtes Netz zum Verstärken der Faserschicht.

Die Erfindung wird nachfolgend anhand der anliegenden Zeichnungen von Ausführungsbeispielen näher erläutert. In den Zeichnungen zeigen:
- Fig. 1: eine Wattescheibe in einer Perspektivansicht schräg von oben;
- Fig. 2: dieselbe Wattescheibe in einem vergrößerten Teilquerschnitt entlang der Linie II-II von Fig. 1;
- Fig. 3: die mit Bindemittel versehene Außenseite derselben Wattescheibe in einer teilweisen Draufsicht;
- Fig. 4: eine Wattescheibe mit einem schuppenförmigen Bindemittelauftrag in einer teilweisen Draufsicht;
- Fig. 5: eine Wattescheibe mit einem netzförmigen Bindemittelauftrag in einer teilweisen Draufsicht;
- Fig. 6: eine Wattescheibe mit einem wellenförmigen Bindemittelauftrag in einer Draufsicht;
- Fig. 7: eine Wattescheibe mit einem anderen wellenförmigen Bindemittelauftrag in einer Draufsicht.

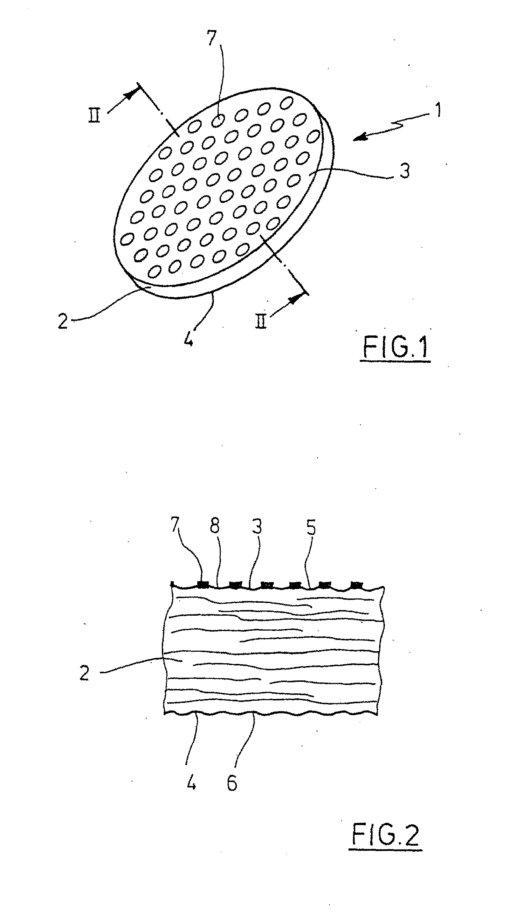

Gemäß Fig. 1 und 2 hat eine ovale Wattescheibe 1 eine Faserschicht 2, die von einem trocken gelegten Vlies aus gebleichten und/oder ungebleichten Baumwollfasern gebildet ist. An den beiden Außenseiten 3, 4 ist die Faserschicht 2 durch Wasserstrahlvernadeln verfestigt. Infolge der Wasserstrahlvernadelung sind an beiden Außenseiten 3, 4 eine Vielzahl feiner Rillen 5, 6 vorhanden.

Auf der Außenseite 3 weist die Faserschicht 2 den Bindemittelauftrag 7 auf, der eine Vielzahl ringförmiger Bereiche umfaßt, die Teilbereiche der Außenseite 3 bedecken. Zwischen den ringförmigen Bereichen des Bindemittelauftrages 7 sind bindemittelfreie Teilbereiche 8 der Außenseite 3 vorhanden.

Bei dem Bindemittelauftrag handelt es sich z.B. um das Produkt NACRYLIC^{™} ABX-30 der Firma Celanese, Bridgewater, NJ 08807, das als wäßrige Emulsion bzw. Dispersion auf die Faserschicht aufgebracht wird. Der Bindemittelauftrag hat ein Flächengewicht von etwa 5 bis 10 g/m².

Die Wattescheibe hat insgesamt ein Flächengewicht von etwa 220 bis 250 g/m².

Der Bindemittelauftrag 7 ist z.B. im Druckverfahren aufgebracht.

Zusätzlich zu dem Bindemittelauftrag 7 in den oberflächennahen Bereichen kann die Faserschicht 2 einen Bindemittelauftrag enthalten, der sich über die gesamte Tiefe der Faserschicht 2 erstreckt. Hierfür kann grundsätzlich dasselbe Material verwendet werden, wie für den Bindemittelauftrag 7. Gegebenenfalls wird dieser weitere Bindemittelauftrag mit einem geringeren Flächengewicht aufgebracht, weil die Faserschicht an der Außenseite stärker belastet wird als in den darunterliegenden Bereichen.

Für das Reinigen und Peeling der Haut wird die Wattescheibe 1 mit der Außenseite 3 auf die Haut aufgesetzt. Im Bereich des Bindemittelauftrages 7 ist die Wattescheibe 1 abrasiv, so daß die Außenseite 3 in der Lage ist, Hautverunreinigungen abzuschürfen. Gleichzeitig werden die abgeschabten Substanzen an den bindemittelfreien Teilbereichen 8 angelagert bzw. von diesen absorbiert und von der Haut entfernt.

Die bindemittelfreie Außenseite 3 kann für das Abnehmen loser Substanzen von der Haut genutzt werden bzw. für eine Endreinigung der Haut.

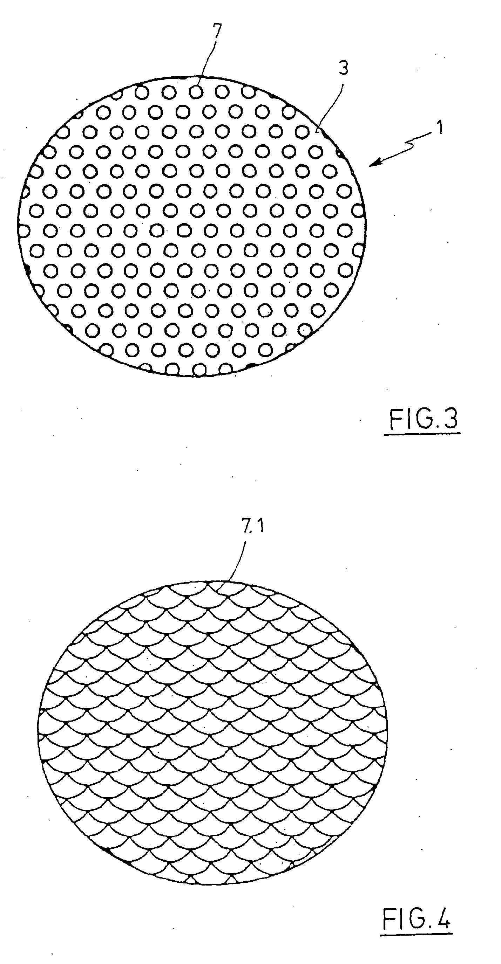

Gemäß Fig. 4 ist der Bindemittelauftrag 7.1 schuppenförmig, d.h. er verläuft entlang der Grenzen einer schuppenartigen Struktur.

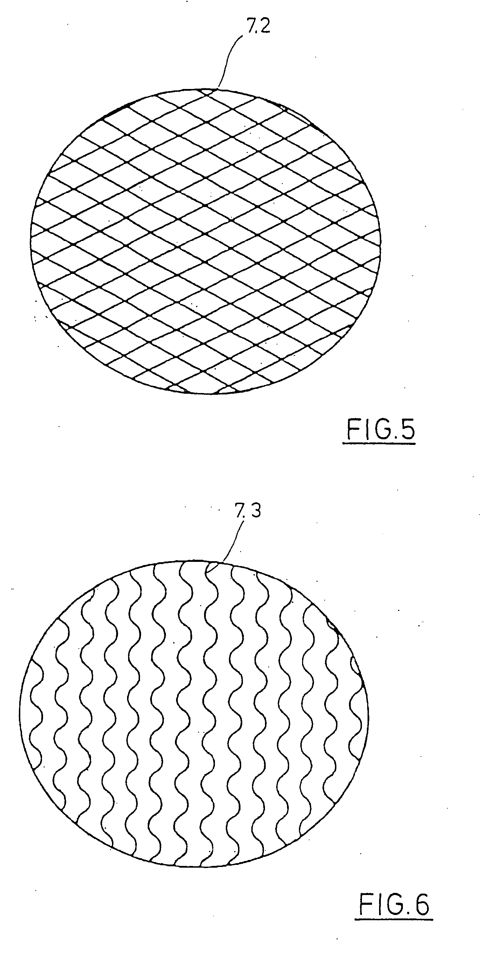

Gemäß Fig. 5 ist der Bindemittelauftrag 7.2 netzförmig, wobei die Linien des Netzes einander und in spitzen Winkeln schneiden.

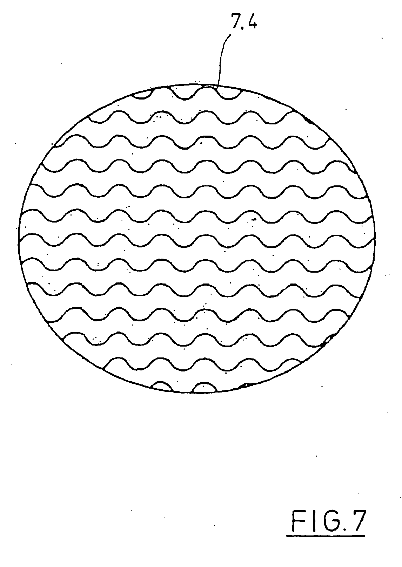

Gemäß Fig. 6 und 7 ist der Bindemittelauftrag 7.3 bis 7.4 wellenförmig, wobei er entlang paralleler Wellenlinien oder -bahnen quer zur Hauptausdehnungsrichtung bzw. in Hauptausdehnungsrichtung der Wattescheibe verläuft.

Die Bindemittelaufträge gemäß Fig. 4 bis 7 können an einer Wattescheibe ausgebildet sein, die im übrigen so ausgebildet ist, wie in den Fig. 1 und 2 gezeigt.

## Patentansprüche

1. Wattescheibe zum Reinigen und Peeling der Haut umfassend mindestens eine Faserschicht (2) und einen abrasiven, mit der Faserschicht verbundenen Bindemittelauftrag (7) an mindestens einer Außenseite (3).

2. Wattescheibe nach Anspruch 1, bei der die Außenseite (3) den Bindemittelauftrag (7) in mindestens einen Teilbereich und mindestens einen weiteren, vom Bindemittelauftrag freien Teilbereich (8) aufweist.

3. Wattescheibe nach Anspruch 1 oder 2, bei dem der Bindemittelauftrag (7) entlang mindestens einer kreisförmigen und/oder wellenlinienförmigen und/oder dreiecksförmigen und/oder schuppenförmigen und/oder gerade und/oder einander kreuzender Linien und/oder Bahnen und/oder einem Logo und/oder einer sonstigen Kennzeichnung und/oder mindestens einem Ornament angeordnet ist.

4. Wattescheibe nach einem der Ansprüche 1 bis 3, bei der der Bindemittelauftrag (7) eine andere Farbe als die Faserschicht (2) aufweist.

5. Wattescheibe nach einem der Ansprüche 1 bis 4, bei der der Bindemittelauftrag als wäßrige Emulsion oder Dispension polymerer Tröpfchen oder Teilchen aufgebracht ist.

6. Wattescheibe nach einem der Ansprüche 1 bis 5, bei der der Bindemittelauftrag (7) ein Acrylpolymer enthält.

7. Wattescheibe nach einem der Ansprüche 1 bis 6, bei der der Bindemittelauftrag (7) partikelfrei ist.

8. Wattescheibe nach einem der Ansprüche 1 bis 7, bei der der Bindemittelauftrag Peelingkörper und/oder Wirkstoffe enthält.

9. Wattescheibe nach einem der Ansprüche 1 bis 8, bei der der Bindemittelauftrag im wesentlichen in die gesamte Tiefe der Faserschicht eingedrungen und mit dieser verbunden ist.

10. Wattescheibe nach einem der Ansprüche 1 bis 9, bei der der Bindemittelauftrag (7) nur in oberflächennahen Bereichen in die Faserschicht (2) eingedrungen und mit dieser verbunden ist.

11. Wattescheibe nach Anspruch 9 und 10, die einen im wesentlichen in die gesamte Tiefe der Faserschicht eingedrungenen und mit dieser verbundenen Bindemittelauftrag und einen weiteren, nur in oberflächennahe Bereiche der Faserschicht eingedrungen und mit dieser verbundenen Bindemittelauftrag aufweist.

12. Wattescheibe nach einem der Ansprüche 1 bis 11, bei der der Bindemittelauftrag (7) im Druck- und/oder Sprüh- und/oder Schaumauftragsverfahren aufgebracht ist.

13. Wattescheibe nach einem der Ansprüche 1 bis 12, bei der der Bindemittelauftrag (7) an der Oberfläche strukturiert ist.

14. Wattescheibe nach einem der Ansprüche 1 bis 13, die ein Flächengewicht von 160 bis 320 g/m2 aufweist.

15. Wattescheibe nach Anspruch 14, die ein Flächengewicht von 220 bis 250 g/m2 aufweist.

16. Wattescheibe nach einem der Ansprüche 1 bis 15, bei der der Bindemittelauftrag (7) ein Flächengewicht von 2 bis 30 g/m2 aufweist.

17. Wattescheibe nach Anspruch 16, bei der der Bindemittelauftrag (7) ein Flächengewicht von 5 bis 10 g/m2 aufweist.

18. Wattescheibe nach einem der Ansprüche 1 bis 17, bei der der Bindemittelauftrag (7) 5 bis 50 % der Außenseite (3) bedeckt.

19. Wattescheibe nach Anspruch 18, bei der der Bindemittelauftrag (7) 10 bis 30 % der Außenseite (3) bedeckt.

20. Wattescheibe nach einem der Ansprüche 1 bis 19, bei der die Faserschicht (2) Baumwollfasern und/oder Viskosefasern und/oder Kunstfasern umfaßt.

21. Wattescheibe nach einem der Ansprüche 1 bis 20, bei der die Faserschicht (2) ein trockengelegtes Vlies ist.

22. Wattescheibe nach einem der Ansprüche 1 bis 21, bei der die Faserschicht (2) wasserstrahlvernadelt und/oder mittels Nadeln mechanisch vernadelt ist.

23. Wattescheibe nach einem der Ansprüche 1 bis 22, bei der die Faserschicht (2) thermoverfestigte Kunststoffasern umfaßt.
